# EUROPEAN PATENT SPECIFICATION

(11) **EP 2 429 661 B1**
(45) Date of publication and mention of the grant of the patent: **18.12.2019**
(21) Application number: 10735309.6
(22) Date of filing: 11.05.2010
(51) Int. Cl.: A62B 18/08

(54) **MASK FOR BREATHING APPARATUS**
ATEMSCHUTZGERÄTEMASKE
MASQUE D'APPAREIL RESPIRATOIRE

(30) Priority: 12.05.2009 GB 0908148
(43) Date of publication of application: 21.03.2012
(73) Proprietor: Scott Health & Safety Ltd., West Pimbo Skelmersdale Lancashire WN8 9RA (GB)
(72) Inventor: SUMNER, Christopher, Lancashire WN8 9RA (GB); THURGOOD, Steven, Lancashire WN8 9RA (GB); LEWIN, Barry, Lancashire WN8 9RA (GB); HENSEY, Paul, Lancashire WN8 9RA (GB); PICKETT, Anthony W., Lancashire WN8 9RA (GB); CARR, Robert, Lancashire WN8 9RA (GB); MARSHALL, Richard D., Lancashire WN8 9RA (GB)
(74) Representative: Hedges, Martin Nicholas
(86) International application number: PCT/GB2010/050768
(87) International publication number: WO 2010/131031

(56) References cited:
- WO-A1-2005/089876
- WO-A1-2007/123585
- US-A- 5 601 078
- US-A1- 2007 240 716
- US-B1- 7 178 931

## Description

The present invention relates to a mask to be used in connection with breathing apparatus which supplies breathable air to the mask. In particular, the present invention is concerned with a single mask which is adapted for connected to both a filtered air supply and a pressurised air supply.

Mask respirators are used in contaminated environments where the air cannot be inhaled for example because the air includes harmful gases or is noxious. Commonly, the mask respirator comprises a mask which has a peripheral seal which seals around the face of the wearer thereby defining a mask space between the mask and face of the wearer and it is from this mask space that air is inhaled by the wearer. The mask includes an inlet for introducing breathable air into the mask space and an outlet for ensuring air exhaled by the wearer is expelled from the mask space. Conventionally, there are two types of air supplies that may be used in combination with this type of mask respirator. The air supply may be sourced from a pressurised air cylinder or the air supply may be sourced from the immediate environment via one or more filters.

It is known in the art to include in breathing masks a system for conveying information to the wearer. Typically, this is achieved by way of a lighting system which switches on or off, or can emit different colors to indicate different events to the wear. In these prior art systems, the lighting system is configured to direct its light towards the eye of the wearer. However, this has been found to be distracting to the wear if it is bright enough to be clearly seen.

US 5,601,078 A1 discloses a mask for breathing apparatus, the mask comprising: a seal for sealing against and around the face of a user; a mask inlet adapted to be placed in fluid communication with a supply of air, a mask outlet (16) through which a user's exhaled breath is emitted.

Other known breathing masks are disclosed in US 7,178,931 B1, WO 2005/089876 A1 and US 2007/0240716 A1.

According to the present invention there is provided a mask for breathing apparatus, the mask comprising a seal for sealing against and around the face of a user; a mask inlet adapted to be placed in fluid communication with a supply of air, a mask outlet through which a user's exhaled breath is emitted, the mask outlet including a one-way exhalation valve including a valve closure member mounted on a valve seat, an oronasal mask in fluid communication with the mask inlet and mask outlet and positioned to engage, in use, over the nose and mouth of the wearer, said oronasal mask being at least partially formed of material which is at least partially translucent, and Illuminating means in optical communication with the oronasal mask such that said illuminating means illuminates said oronasal mask.

A mask in accordance with the invention has the advantage that the illumination of the oronasal mask can be achieved in a more diffuse manner than the direct illumination of the eye of the prior art systems whilst still being easily visible to the wearer. In this way, the information conveyed by the illuminating means is easily discerned by the wearer without being distracting. Furthermore, the at least partially translucent nature of the oronasal mask will make more of the face of the wearer visible to other persons, which has been found to make the appearance of the wearer less alarming, particularly in emergency situations.

The reference above to the oronasal mask being formed of material which is at least partially translucent is intended to cover the mask being formed of material which may be translucent or completely transparent, but not opaque. The oronasal mask may be only partially formed of said translucent / transparent material but is preferably completely formed of said material so as to maximise visibility of the wearer's face and also to make illumination of the oronasal mask as obvious to the wearer as possible.

The illuminating means is preferably removably mounted to the mask, in particular carried on a plug which is removably connectable to a port formed in the mask. The illuminating means may particularly advantageously be a multi-colour LED.

An embodiment of the present invention will now be described by way of example only with reference to the accompanying drawings, in which:
Fig. 1 is a view of a mask for use with breathing apparatus;
Fig. 2 is a diagrammatic partial view of an exhalation valve of a mask which does not form part of the present invention;

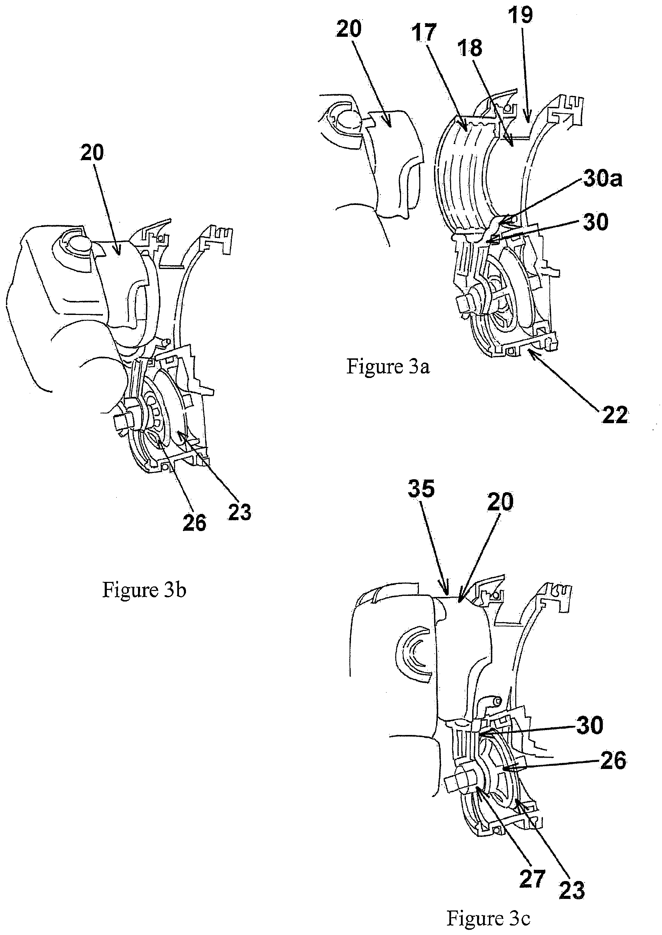

Figs. 3a, 3b and 3c illustrate the attachment of a pressured air supply to the air inlet of the mask of figure 2;
Figure 4 is a perspective view of a mask with a translucent oronasal mask in accordance with the invention; and
Figures 5a to 5c are various views showing illuminating means for use with the translucent oronasal mask of Figure 4.

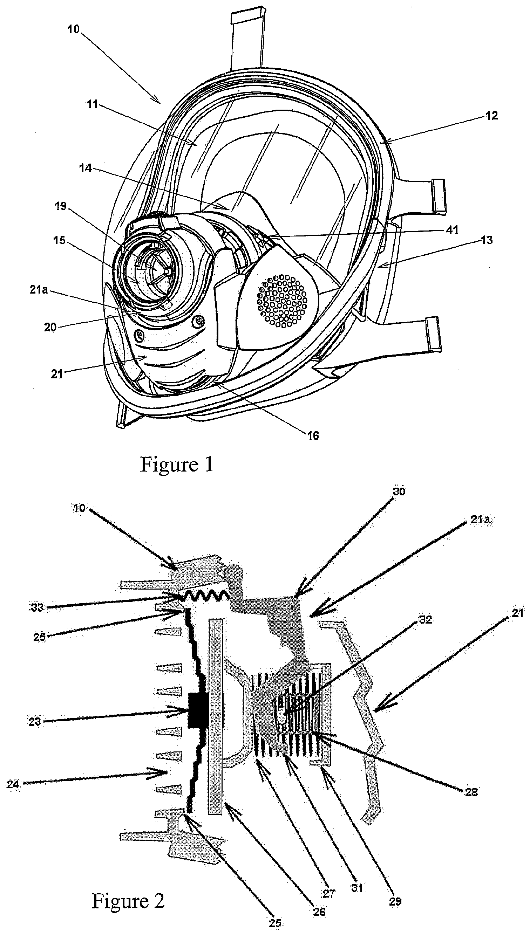

Referring to the figures, the mask 10 shown in Fig. 1 generally comprises a visor 11 in a frame 12 which includes a peripheral seal 13 and means (not shown) for mounting the mask 10 over the face of a user. The peripheral seal 13 is provided to form with the face of a user a substantially airtight seal to prevent ingress of contaminated air from the environment into the enclosed space defined by the mask and the user's face. In this embodiment the mask 10 additionally includes an inner oronasal mask 14 which is intended to fit snugly over the mouth and nose of the mask user. The oronasal mask 14 is in communication with a mask inlet 15 and a mask outlet 16.

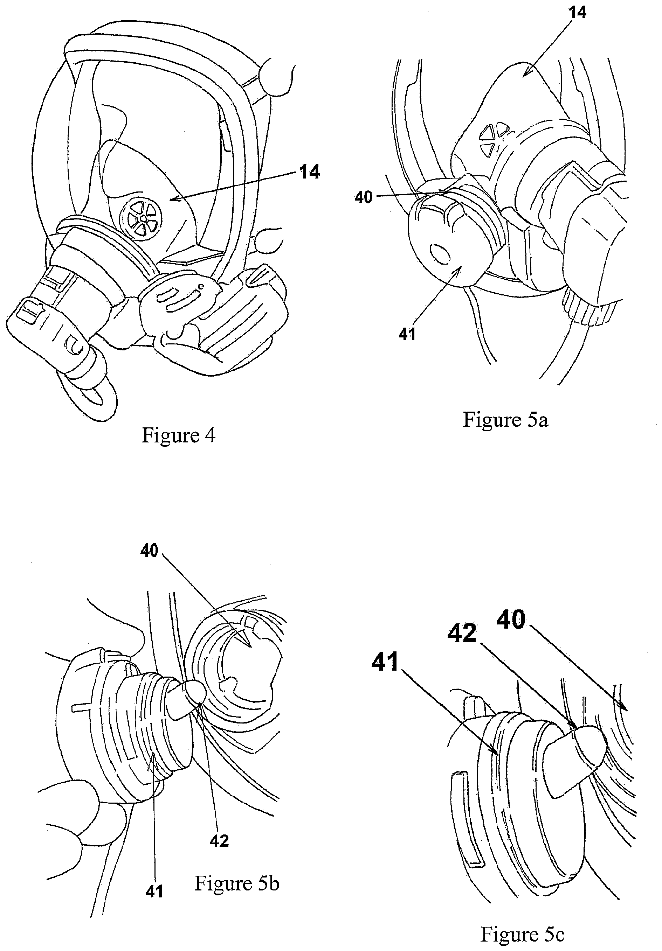

Figures 4 through 5c, shows, in more detail, a mask embodying the present invention. As can clearly been seen in Figure 4, the oronasal mask 14 is formed of translucent material as compared with prior art systems which are formed of opaque material.

The mask furthermore includes a port 40 by means of which an illuminating means 42 - in the illustrated embodiment a 3 colour LED, may be secured to the mask in optical communication with the oronasal mask 14. As illustrated in Figures 5b and 5c, the LED is mounted on a plug 41 which locks in the port 40 in an air tight fashion and has a wire coming out of the back for connection to a power supply, although it will be understood that the plug 41 may also have a self-contained power supply such as a rechargeable battery. It will also be understood that the illuminating means may be integrated directly into the mask instead of being mounted separately thereto.

As already stated, the LED is positioned to emit its light onto the oronasal mask 14. As a result of the translucent nature of the oronasal mask, the light from the LED illuminates the whole of the oronasal mask 14 in a manner which is easily visible to the wearer. As a result, the information conveyed by the activation and/or colour of the LED is easily detected by the wearer since the change in illumination and/or colour of the oronasal mask will be within the normal field of vision and hence discretely visible to the wear. It will, of course, be understood that the oronasal mask may be transparent rather than translucent within the scope of the invention

## Claims

1. A mask (10) for breathing apparatus, the mask comprising:
a seal (13) for sealing against and around the face of a user;
a mask inlet (15) adapted to be placed in fluid communication with a supply of air,
a mask outlet (16) through which a user's exhaled breath is emitted, the mask outlet (16) including a one-way exhalation valve (22) including a valve closure member (23) mounted on a valve seat (25), and
an oronasal mask (14) in fluid communication with the mask inlet (15) and mask outlet (16) and positioned to engage, in use, over the nose and mouth of the wearer, **characterised in that** said oronasal mask (14) is at least partially formed of material which is at least partially translucent, and **in that**
said mask includes illuminating means (42) in optical communication with the oronasal mask (14) such that said illuminating means (42) illuminates said oronasal mask (14).

2. A mask as claimed in claim 1, wherein the whole oronasal mask (14) is formed of material which is at least partially translucent.

3. A mask as claimed in claim 1 or claim 2, wherein the illuminating means (42) is removably mounted to the mask (10).

4. A mask as claimed in claim 3, wherein the illuminating means (42) is carried on a plug (41) which is removably connectable to a port (40) formed in the mask (10).

5. A mask as claimed in any of the preceding claims, wherein the illuminating means (42) is a multi-colour LED.

## Patentansprüche

1. Eine Maske (10) für ein Atemschutzgerät, wobei die Maske umfasst:
eine Dichtung (13) zum Abdichten gegen und um das Gesicht eines Benutzers;
einen Maskeneinlass (15), der geeignet ist, um mit einer Luftzufuhr in Fluidverbindung angeordnet zu werden,
einen Maskenauslass (16), durch welchen ein ausgeatmeter Atem eines Benutzers ausgelassen wird,
wobei der Maskenauslass (16) ein Einweg-Ausatmungsventil (22), einschließend ein Ventilschließelement (23), das an einem Ventilsockel (25) angebracht ist, und
eine oronasale Maske (14) in Fluidverbindung mit dem Maskeneinlass (15) und dem Maskenauslass (16), die so positioniert ist, dass sie bei der Verwendung über Nase und Mund des Trägers anliegt, **dadurch gekennzeichnet, dass** die oronasale Maske (14) zumindest teilweise aus einem Material gebildet ist, das zumindest teilweise durchsichtig ist, und **dadurch, dass** die Maske ein Leuchtmittel (42) einschließt, das derart in optischer Kommunikation mit der oronasalen Maske (14) ist, dass das Leuchtmittel (42) die oronasale Maske (14) beleuchtet.

2. Eine Maske nach Anspruch 1, wobei die gesamte oronasale Maske (14) aus einem Material gebildet ist, das zumindest teilweise durchsichtig ist.

3. Eine Maske nach Anspruch 1 oder Anspruch 2, wobei das Beleuchtungsmittel (42) entfernbar an der Maske (10) angebracht ist.

4. Eine Maske nach Anspruch 3, wobei das Beleuchtungsmittel (42) auf einem Stecker (41) getragen wird, der lösbar mit einem in der Maske (10) ausgebildeten Anschluss (40) verbindbar ist.

5. Eine Maske nach einem der vorhergehenden Ansprüche, wobei das Beleuchtungsmittel (42) eine mehrfarbige LED ist.

## Revendications

1. Masque (10) pour un appareil respiratoire, le masque comprenant :
un joint (13) pour assurer une étanchéité contre et autour du visage d'un utilisateur ;
une entrée de masque (15) conçue pour être placée en communication fluidique avec une alimentation en air,
une sortie de masque (16) à travers laquelle le souffle expiré d'un utilisateur est émis, la sortie de masque (16) incluant une soupape d'expiration unidirectionnelle (22) incluant un élément de fermeture de soupape (23) monté sur un siège de soupape (25), et
un masque oro-nasal (14) en communication fluidique avec l'entrée de masque (15) et la sortie de masque (16) et positionné pour venir se placer, en cours d'utilisation, par-dessus le nez et la bouche du porteur, **caractérisé en ce que** ledit masque oro-nasal (14) est au moins partiellement formé d'un matériau qui est au moins partiellement translucide, et **en ce que** ledit masque inclut un moyen d'éclairage (42) en communication optique avec le masque oro-nasal (14) de telle sorte que ledit moyen d'éclairage (42) éclaire ledit masque oro-nasal (14).

2. Masque selon la revendication 1, dans lequel le masque oro-nasal entier (14) est formé d'un matériau qui est au moins partiellement translucide.

3. Masque selon la revendication 1 ou la revendication 2, dans lequel le moyen d'éclairage (42) est monté de manière amovible sur le masque (10).

4. Masque selon la revendication 3, dans lequel le moyen d'éclairage (42) est supporté sur un bouchon (41) qui peut être raccordé de façon amovible à un orifice (40) formé dans le masque (10).

5. Masque selon l'une quelconque des revendications précédentes, dans lequel le moyen d'éclairage (42) est une DEL multicolore.
